(19) Europäisches Patentamt
European Patent Office
Office européen des brevets

(11) EP 4 360 638 B1

(12) **EUROPEAN PATENT SPECIFICATION**

(45) Date of publication and mention of the grant of the patent:
**12.11.2025 Bulletin 2025/46**

(21) Application number: **22836835.3**

(22) Date of filing: **04.07.2022**

(51) International Patent Classification (IPC):
***A61K 31/737*** (2006.01) ***A61P 7/02*** (2006.01)
***C08B 37/00*** (2006.01) ***C08L 5/00*** (2006.01)

(52) Cooperative Patent Classification (CPC):
**C08B 37/006; A61K 31/737; A61P 7/02; C08L 5/00;** Y02A 50/30

(86) International application number:
**PCT/CN2022/103538**

(87) International publication number:
**WO 2023/280084 (12.01.2023 Gazette 2023/02)**

(54) **APPLICATION OF SULFATE GLUCO-GALACTO-OLIGOSACCHARIDE IN PREPARATION OF ANTICOAGULANT AND/OR ANTITHROMBOTIC DRUG**

VERWENDUNG VON SULFATGLUCO-GALACTO-OLIGOSACCHARID BEI DER HERSTELLUNG EINES ANTIKOAGULANS UND/ODER EINES ANTITHROMBOTIKUMS

APPLICATION DE GLUCO-GALACTO-OLIGOSACCHARIDE DE SULFATE DANS LA PRÉPARATION D'UN MÉDICAMENT ANTICOAGULANT ET/OU ANTITHROMBOTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.07.2021 CN 202110779428**
**04.11.2021 CN 202111299660**

(43) Date of publication of application:
**01.05.2024 Bulletin 2024/18**

(73) Proprietor: **Nanjing Kangbo Biotechnology Co., Ltd.**
**Nanjing City, Jiangsu Province 211800 (CN)**

(72) Inventors:
- **ZHANG, Jianfa**
  **Nanjing, Jiangsu 210094 (CN)**
- **LIN, Qian**
  **Nanjing, Jiangsu 210094 (CN)**
- **WANG, Lei**
  **Nanjing, Jiangsu 210094 (CN)**

(74) Representative: **De Clercq & Partners**
**Edgard Gevaertdreef 10a**
**9830 Sint-Martens-Latem (BE)**

(56) References cited:
**CN-A- 1 251 992** **CN-A- 101 209 260**
**CN-A- 113 440 540** **CN-A- 113 633 658**
**CN-A- 114 533 756** **US-A- 6 143 730**

- **MARTINICHEN-HERRERO, J.C. ; CARBONERO, E.R. ; SASSAKI, G.L. ; GORIN, P.A.J. ; IACOMINI, M.: "Anticoagulant and antithrombotic activities of a chemically sulfated galactoglucomannan obtained from the lichen Cladoniaibitipocae", INTERNATIONAL JOURNAL OF BIOLOGICAL MACROMOLECULES, ELSEVIER BV, NL, vol. 35, no. 1-2, 1 March 2005 (2005-03-01), NL , pages 97 - 102, XP027766718, ISSN: 0141-8130**
- **WANG LEI, CHENG RUI, SUN XIAQING, ZHAO YANG, GE WENHAO, YANG YUNXIA, GAO YAN, DING ZHAO, LIU JUNHAO, ZHANG JIANFA: "Preparation and Gut Microbiota Modulatory Property of the Oligosaccharide Riclinoctaose", JOURNAL OF AGRICULTURAL AND FOOD CHEMISTRY, AMERICAN CHEMICAL SOCIETY, US, vol. 69, no. 12, 31 March 2021 (2021-03-31), US , pages 3667 - 3676, XP055940823, ISSN: 0021-8561, DOI: 10.1021/acs.jafc.0c07783**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 360 638 B1

## Description

### TECHNICAL FIELD

The present disclosure relates to the technical field of anticoagulants and antithrombotic drugs, and specifically relates to use of sulfate gluco-galacto-oligosaccharide in preparation of an anticoagulant and/or antithrombotic drug.

### BACKGROUND

Blood coagulation refers to a process in which blood changes from a flowing liquid into an immobile gel, and is essentially a process in which water-soluble fibrinogen transforms into water-insoluble solid fibrin. This process includes the generation of a prothrombin activator under the intrinsic or/and extrinsic coagulation pathway, the generation of thrombin under the action of a coagulation factor, and the conversion of fibrinogen into fibrin under the action of the thrombin.

Thrombus is a small piece of blood formed on a surface of the inner surface of the blood vessel of the cardiovascular system where it is peeled off or repaired. Thrombus formation involves local blood coagulation in the vasculature and generally leads to serious illnesses such as heart attacks and strokes. Risk factors for thrombosis include abnormal hyperlipidemia, hyperglycemia, and elevated plasma fibrinogen.

Heparin is the most widely used anticoagulant drug that has been in clinical use for more than 90 years. The heparin is a mucopolysaccharide extracted from the intestinal mucosa or liver of pigs, sheep, cattle and other animals, and to a certain extent results in a waste of animal resources. Heparin can prevent and treat thrombosis, but can also produce side effects, such as easily causing spontaneous bleeding, manifested as various mucosal bleeding, joint bleeding, and wound bleeding. After the heparin is injected, a number of platelets in the blood decreases significantly. Long-term use of the heparin can lead to a decrease in bone mineral density, causing osteoporosis and increasing the risk of fractures. Low-molecular-weight heparin is produced by controlled depolymerization of the heparin. Compared with unfractionated heparin, the low-molecular-weight heparin shows higher availability and longer half-life, and has become the anticoagulant drug of choice for the treatment of deep vein thrombosis. However, low-molecular-weight heparin is essentially derived from animal tissues, and its anticoagulant effect is difficult to reverse, leading to the risk of bleeding due to overdose. Ultra-low-molecular-weight heparin is mainly a type of chemically synthesized heparinoid with desirable pharmacokinetic control and no contamination by viral impurities. However, these drugs are complex in chemical synthesis with an extremely high cost, and their anticoagulant effects are also not easily reversed. Therefore, there is an urgent clinical need to find novel antithrombotic drugs and anticoagulants with fewer side effects than those of the heparin.

Previous studies have reported a gluco-galacto-oligosaccharide and a preparation method thereof, where the gluco-galacto-oligosaccharide has a definite molecular weight, a low viscosity, and a desirable water solubility. US6143730A and CN1251992A describe sulfated oligosaccharide compounds of formula R1-(Rx)n-R2 having anticoagulant/antithrombotic activity, wherein n = 1 -6, R1, Rx and R2 may represent the same of different monosaccharide units (e.g. glucose, and galactose) connected via 1-→2, 1-3, 1-→4 or 1-→6 glycosidic bonds. CN101209260A discloses sulfated polysaccharides (particularly sulfated inulin) having anticoagulant activity. Martinichen-Herrero et al. report the anticoagulant and antithrombotic activity of sulfated galactoglucomannan (Anticoagulant and antithrombotic activities of a chemically sulfated galactoglucomannan obtained from the lichen Cladonia ibitipocae, International Journal of Biological Macromolecules, vol. 35, pages 97-102). Wang et al. describe the activity of the octasaccharide riclinoctaose (Preparation and Gut Microbiota Modulatory Property of the Oligosaccharide Riclinoctaose, Journal of Agricultural and Food Chemistry, vol. 69, pages 3667-3676). Moreover, it is pointed out that the gluco-galacto-oligosaccharide, as a prebiotic, exhibits a biological activity to significantly improve the gut microbiota (Wang, L., Cheng, R., Sun, X., et al. Preparation and Gut Microbiota Modulatory Property of the Oligosaccharide Riclinoctaose, J. Agric. Food Chem. 2021, Mar 31, 69(12): 3667-3676.). International patent WO2019090203A1 has disclosed a sulfated heparan sulfate oligosaccharide compound with an anti-inflammatory activity, but the compound does not have anticoagulant activity. The above examples indicate that oligosaccharides do not necessarily have anticoagulant and antithrombotic activities after sulfation modification.

### SUMMARY

An objective of the present disclosure is to provide an anticoagulant and/or antithrombotic drug, wherein the anticoagulant and/or antithrombotic drug comprises sulfate gluco-galacto-oligosaccharide and the sulfate gluco-galacto-oligosaccharide has a structural formula shown in formula 1:

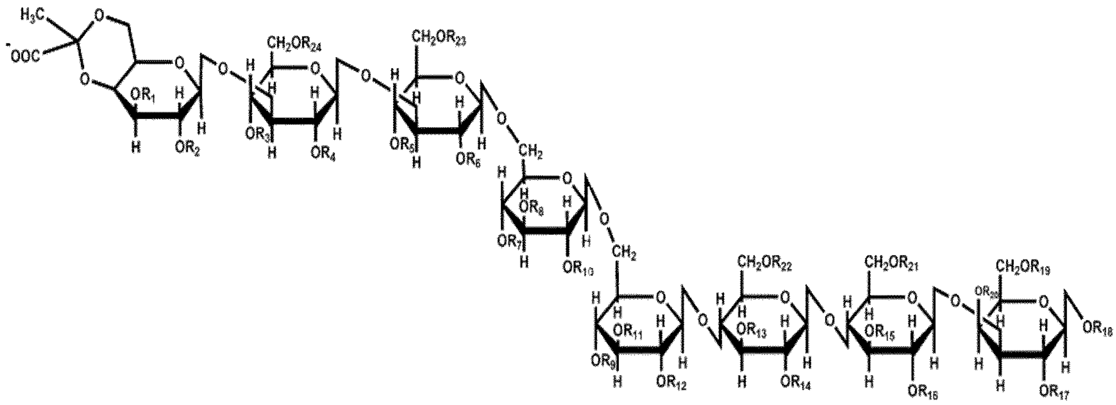

formula 1,

wherein, $R_1$, $R_2$, $R_3$, $R_4$, $R_5$, $R_6$, $R_7$, $R_8$, $R_9$, $R_{10}$, $R_{11}$, $R_{12}$, $R_{13}$, $R_{14}$, $R_{15}$, $R_{16}$, $R_{17}$, $R_{18}$, $R_{19}$, $R_{20}$, $R_{21}$, $R_{22}$, $R_{23}$, and $R_{24}$ independently are selected from the group consisting of $SO_3^-$ and H, but are not all H, and wherein the sulfate gluco-galacto-oligosaccharide has an average of 0.5 to 3 $SO_{3-}$ on each sugar residue.

In the present disclosure, the sulfate gluco-galacto-oligosaccharide has a high activity in inhibiting the production of thrombin by coagulation factors, and can be used as an anticoagulant or antithrombotic drug to control thrombus.

The present disclosure further provides the anticoagulant and/or antithrombotic drug for use in the treatment of cerebral infarction, atherosclerotic plaque formation in a carotid artery, pulmonary infarction, myocardial infarction, splenic infarction, thrombosis in a mesenteric vein, mesenteric arterial embolism, a venous thromboembolic disease, lower extremity arteriosclerosis obliterans and disseminated intravascular coagulation, or for use as an anticoagulant of a blood sample specimen for blood transfusion, extracorporeal hemodialysis, or peritoneal dialysis.

In the present disclosure, the greater the average number of $SO_3^-$ on each sugar residue, the higher the inhibition rate of FXa. In a specific example, there are preferably 0.5 to 2.5, more preferably 2.1 to 2.5 $SO_3^-$ on average per sugar residue.

In the present disclosure, the sulfate gluco-galacto-oligosaccharide has 8 sugar units, including 7 glucose residues and 1 galactose residue. A 1st glucose residue is ligated to a pyruvate group, while an 8th sugar unit is a galactose residue. A specific ligation method of the 7 glucose residues (Glcp) and the 1 galactose residue (Galp) is shown in formula 2:

D-Glcp(4,6-pyr)-β-(1→3)-D-Glcp-β-(1→3)-D-Glcp-β-(1→6)-D-Glcp-β-(1→6)-D-Glcp-β-(1→4)-D-Glcp-β-(1→4)-D-Glcp-β-(1→3)-D-Galp, formula 2.

In the present disclosure, a preparation method the sulfate gluco-galacto-oligosaccharide includes: subjecting gluco-galacto-oligosaccharide to sulfation with a sulfating reagent to obtain the sulfate gluco-galacto-oligosaccharide.

In the present disclosure, a sulfation degree of the gluco-galacto-oligosaccharide can be controlled by controlling a ratio of the gluco-galacto-oligosaccharide and the sulfating reagent, a reaction temperature, and a reaction time. That is, an average number of sulfate groups on each sugar residue is controlled to obtain gluco-galacto-oligosaccharide types substituted with sulfate groups to varying degrees.

In the present disclosure, the sulfating reagent is a sulfating reagent commonly used in this field, as long as it can sulfate the gluco-galacto-oligosaccharide. In a specific example, the sulfating reagent is preferably a mixture of pyridine and chlorosulfonic acid.

In a specific example, the preparation method of the sulfate gluco-galacto-oligosaccharide specifically includes: preparing the sulfating reagent using pyridine and chlorosulfonic acid; adding a dimethyl sulfoxide (DMSO) solution of the gluco-galacto-oligosaccharide into the sulfating reagent under stirring to allow the sulfation in an oil bath at 50°C to 120°C; and subjecting an obtained reaction solution to a post-treatment and purification to obtain the sulfate gluco-galacto-oligosaccharide.

Preferably, the pyridine and the chlorosulfonic acid in the mixture are at a volume ratio of 3:1 to 8:1.

Preferably, the gluco-galacto-oligosaccharide and DMSO in the DMSO solution of the gluco-galacto-oligosaccharide are at a mass-to-volume ratio of 1 g: (10-20) mL.

Preferably, the gluco-galacto-oligosaccharide and the sulfating reagent are at a mass-to-volume ratio of 1 g: 20 mL to 1 g: 60 mL.

Preferably, the sulfation is conducted at 60°C.

Preferably, the sulfation is conducted for 2 h to 5 h.

Preferably, the post-treatment and the purification include: adjusting the reaction solution to a neutral pH value with a 15% NaOH solution, conducting centrifugation at 8,000 rpm and 4°C to remove insoluble impurities, collecting an obtained supernatant to allow dialysis with a dialysis bag of 500 Da to 1,000 Da, subjecting a resulting dialyzate to concentration and desalination using an organic ultrafiltration membrane, and then conducting freeze-drying or ethanol precipitation to obtain the sulfate gluco-galacto-oligosaccharide.

Compared with the prior art, the present disclosure has the following advantages:

In the present disclosure, it is discovered for the first time that the sulfate gluco-galacto-oligosaccharide has obvious anticoagulant activity *in vitro,* and can selectively inhibit a coagulation factor Xa (FXa), resulting in a small risk of bleeding. Therefore, the sulfate gluco-galacto-oligosaccharide is suitable for preparing *in vitro* anticoagulants. At the same time, the sulfate gluco-galacto-oligosaccharide exhibits a function of inhibiting thrombosis in mouse models and can be used to control thrombosis. The sulfate gluco-galacto-oligosaccharide is safe and non-toxic, has a strong medicinal effect, and shows broad application prospects in the anticoagulant and/or antithrombotic drug.

## BRIEF DESCRIPTION OF THE DRAWINGS

FIG. 1 shows results of an inhibitory activity of the sulfate gluco-galacto-oligosaccharide against FXa, including the inhibitory activities of gluco-galacto-oligosaccharide and sulfate gluco-galacto-oligosaccharide against FXa, both at a concentration of 1 mg/mL;

FIG. 2 shows an effect of the sulfate gluco-galacto-oligosaccharide on activated partial thromboplastin time (APTT), including the effects of sulfate gluco-galacto-oligosaccharide samples of three different concentrations on plasma coagulation time;

FIG. 3 shows that the sulfate gluco-galacto-oligosaccharide inhibits the formation of arterial thrombosis induced by $FeCl_3$, where a group (a) is the thrombosis of mice injected with physiological saline; a group (b) is the thrombosis of mice injected with 0.5 mg/mL sulfate gluco-galacto-oligosaccharide; and a group (c) is the thrombosis of mice injected with 1.0 mg/mL sulfate gluco-galacto-oligosaccharide; and

FIG. 4 shows an effect of the sulfate gluco-galacto-oligosaccharide on a tail bleeding time of mice, including the effects of sulfate gluco-galacto-oligosaccharide samples of two different concentrations on the bleeding situation at the tail tip of mice.

## DETAILED DESCRIPTION OF THE EMBODIMENTS

Unless otherwise specified, all technical and scientific terms used in the present disclosure have the same meaning as commonly understood by one of ordinary skill in the technical field to which the present disclosure belongs. Unless otherwise specified, the reagents or materials used in the following examples can be purchased commercially or synthesized by referring to existing methods.

In the present disclosure, the gluco-galacto-oligosaccharide is prepared by conventional methods known in the art. Specifically, the gluco-galacto-oligosaccharide can be prepared according to a method taught in: Wang, L., Cheng, R., Sun, X. et al., J. Agric. Food Chem., 69, pp. 3667-3676 (2021).

In the present disclosure, the term "and/or" means use alone or in combination.

The present disclosure is described in more detail below with reference to the examples and accompanying drawings.

### Example 1

Preparation of sulfate gluco-galacto-oligosaccharide:

1.0 g of gluco-galacto-oligosaccharide was transferred to an Erlenmeyer flask, added with 10 mL to 20 mL of DMSO, and dissolved by stirring to obtain a gluco-galacto-oligosaccharide solution. Pyridine and chlorosulfonic acid were mixed in a three-necked flask at a volume ratio of 3:1, and then magnetically stirred in an ice-water bath for 30 min to obtain a sulfating reagent. The gluco-galacto-oligosaccharide solution was gradually added into 30 mL of the sulfating reagent under stirring, a condenser tube was connected while the other two bottle caps were plugged, and the three-necked flask was placed in an oil bath at 60°C to allow a reaction by stirring for 3 h. After naturally cooling to a room temperature, the reaction was stopped. A reaction solution was adjusted to a neutral pH value with a 15% NaOH solution, centrifugation was conducted at 8,000 rpm and 4°C to remove insoluble impurities, an obtained supernatant was collected to allow dialysis with a dialysis bag of 500 Da to 1,000 Da, a resulting dialyzate was subjected to concentration and desalination using an organic ultrafiltration membrane, and then freeze-drying or ethanol precipitation was conducted to obtain the sulfate gluco-galacto-oligosaccharide with averagely 1.65 sulfate groups per sugar residue.

According to the above preparation method, the gluco-galacto-oligosaccharide and the sulfating reagent were subjected to sulfation at a mass-to-volume ratio (g: mL) of 1:20, 1:30, 1:45, and 1:60, to obtain sulfate gluco-galacto-oligosaccharide samples with degrees of substitution (i.e., the average number of sulfate groups on each sugar residue) of 1.23, 1.65, 2.10, and 2.52, respectively.

Determination of the degree of sulfation substitution (Ds) of sulfate gluco-galacto-oligosaccharide

200 μL of 10 mg/mL sulfate gluco-galacto-oligosaccharide solution was added into a glass bottle, added with an equal volume of 4 M HCl, and complete acid hydrolysis was conducted at 100°C for 8 h, and a resulting product was blown dry with nitrogen and then redissolved with 100 μL of pure water. A 0.5% gelatin solution was prepared and added with 0.5% $BaCl_2$ when using. 100 μL of the sulfate gluco-galacto-oligosaccharide solution was mixed with an equal volume of $BaCl_2$ and the gelatin solution, and an OD value was measured at 360 nm. The Ds was calculated by the following equation 1:

$$DS = \frac{162 * S\%}{32 - 102 * S\%} \text{ equation 1,}$$

The dosage of sulfating reagent, reaction time, reaction temperature and other conditions all affected the degree of sulfation substitution. The dosage of sulfating reagent was reduced, and the degree of sulfation substitution could also be reduced.

In Examples 2 to 5, unless otherwise specified, the sulfate gluco-galacto-oligosaccharide was the sulfate gluco-galacto-oligosaccharide with averagely 1.65 sulfate groups on each sugar residue.

**Example 2**

Determination of the inhibitory activity of sulfate gluco-galacto-oligosaccharide on coagulation factor FXa

1. Determination of inhibitory activity

a. 100 μL of FXa (5 nM) and 50 μL of the sulfate gluco-galacto-oligosaccharide sample (1 mg/mL) were mixed in a 96-well plate, incubated at 37°C for 1 h, added with 50 μL of a substrate CS-11(22) (1.77 mM), and detected at a wavelength of 405 nm for 60 min, detecting once every minute.

b. 100 μL of HFXIa (human coagulation factor XIa, 1 nM) and 50 μL of the sulfate gluco-galacto-oligosaccharide sample (1 mg/mL) were mixed in a 96-well plate, incubated at 37°C for 1 h, added with 50 μL of a substrate S2366 (1 mM), and detected at a wavelength of 405 nm for 60 min, detecting once every minute.

c. 100 μL of FXIIa (4nM) and 50 μL of the sulfate gluco-galacto-oligosaccharide sample (1 mg/mL) were mixed in a 96-well plate, incubated 37°C for 1 h, added with 50 μL of a substrate Pefachrome FXIIa/TH5253 (1 mM), and detected at a wavelength of 405 nm for 60 min, detecting once every minute.

d. 100 μL of Trypsin (8.3 nM) and 50 μL of the sulfate gluco-galacto-oligosaccharide sample (1 mg/mL) were mixed in a 96-well plate, incubated 37°C for 1 h, added with 50 μL of a substrate CS-11(22) (1 mM), and detected at a wavelength of 405 nm for 60 min, detecting once every minute.

e. 100 μL of Thrombin (0.2 IU/mL) and 50 μL of the sulfate gluco-galacto-oligosaccharide sample (1 mg/mL) were mixed in a 96-well plate, incubated at 37°C for 1 h, added with 50 μL of a substrate CS-01(38) (400 μM), and detected at a wavelength of 405 nm for 60 min, detecting once every minute.

f. 100 μL of Kallikrein (1 nM) and 50 μL of the sulfate gluco-galacto-oligosaccharide sample (1 mg/mL) were mixed in a 96-well plate, incubated 37°C for 1 h, added with 50 μL of a substrate CS-31(02) (1 mM), and detected at a wavelength of 405 nm for 60 min, detecting once every minute.

2. Data analysis

Data processing was conducted using the software Graphpad Prism 6.0. Regression analysis was conducted with the reaction time as an abscissa and the absorbance value at 405 nm as an ordinate.

The inhibitory activity of sulfate gluco-galacto-oligosaccharide against other enzymes was measured using a

chromogenic substrate method to determine whether the sulfate gluco-galacto-oligosaccharide had a selective specificity for FXa. As shown in Table 1, the sulfate gluco-galacto-oligosaccharide had the selective specificity for FXa but had no inhibitory activity against other coagulation factors.

Table 1 Inhibitory effects of sulfate gluco-galacto-oligosaccharide on different coagulation factors

| Coagulation factor | Inhibitory effect |
|---|---|
| Coagulation factor FXa | Inhibition |
| Coagulation factor FXIa | No inhibition |
| Coagulation factor FXIIa | No inhibition |
| Trypsin | No inhibition |
| Thrombin | No inhibition |
| Kallikrein | No inhibition |

An absorbance value-reaction time curve was plotted with data points generated by a microplate reader, where the slope was a speed V of the enzymatic reaction, the negative control group had a reaction speed V0, and the sample had a reaction speed Vi. The calculated inhibition rate = (V0-Vi)/V0×100%. According to FIG. 1, when sulfate gluco-galacto-oligosaccharide with averagely 1.65 sulfate groups per sugar residue was at 1 mg/mL, the inhibition rate of FXa was 57.6%. This indicated that the sulfate gluco-galacto-oligosaccharide had the inhibitory activity on FXa, while the gluco-galacto-oligosaccharide had no inhibitory activity on FXa. The degree of sulfation of the sulfate gluco-galacto-oligosaccharide was controlled by controlling the ratio of gluco-galacto-oligosaccharide and sulfating reagent. Table 2 showed the inhibitory activity of sulfate gluco-galacto-oligosaccharide samples with different sulfation degrees on FXa. It was seen that the more sulfate groups on average per sugar residue, the higher the inhibition rate of FXa by the sulfate gluco-galacto-oligosaccharide.

Table 2 Inhibition rate of FXa by sulfate gluco-galacto-oligosaccharide samples with different sulfation degrees

| Gluco-galacto-oligosaccharide: sulfating reagent (m:v, g:mL) | Average number of sulfate groups per sugar residue | Inhibition rate |
|---|---|---|
| 1:10 | 0.47 | 10.2% |
| 1:20 | 1.23 | 39.2% |
| 1:30 | 1.65 | 57.6% |
| 1:45 | 2.10 | 65.5% |
| 1:60 | 2.52 | 78.4% |

**Example 3**

Effect of sulfate gluco-galacto-oligosaccharide on coagulation time

1. Establishment of a mouse model:

The experimental animals were male C57/BL-6J mice, weighing 18 g to 20 g. The mice were housed under standard experimental conditions with a 12-hour light-12-hour dark cycle with free access to water and food. The mice were anesthetized by intraperitoneal injection of 5% chloral hydrate, and administration of the drug was administered through the tail vein after confirming that the mice were completely anesthetized and had no eyelid reflex. The mice were randomly divided into three groups. A first group was a negative control group and was injected with physiological saline through the tail vein; a second group was a low-dose group, with 0.25 mg/mL sulfate gluco-galacto-oligosaccharide injected into the tail vein; a third group was a medium-dose group, with 0.5 mg/mL sulfate gluco-galacto-oligosaccharide injected into the tail vein; and a fourth group was a high-dose group, with 1.0 mg/mL sulfate gluco-galacto-oligosaccharide injected into the tail vein. Blood was collected from the abdominal aorta of mice and placed in silanized tubes moistened with anticoagulant, and then incubated at 37°C.

2. Determination of ATPP

Activated partial thromboplastin time (ATPP) is a duration required to observe plasma coagulation after adding an APTT reagent and calcium ions to the tested plasma. The ATPP mainly reflects the condition of the endogenous coagulation system. Whole blood was collected and diluted appropriately, centrifuged at 1,080 rpm for 10 min, and an upper layer of platelet-rich plasma was removed. After centrifugation at 3,000 rpm for 5 min, a platelet-poor plasma (PPP) in the upper layer was taken for coagulation time measurement. The ATPP was measured according to the operating procedures in the kit instructions, and the fibrin formation time was read on the coagulometer.

3. Data analysis

Data processing was conducted using the Graphpad Prism 6.0 software. An APTT line chart was plotted with the sample concentration as an abscissa and the plasma coagulation time multiple as an ordinate. $P<0.05$ indicated a significant difference.

The coagulation time of the physiological saline group was recorded as 1 times, and the multiples of APTT of the sulfate gluco-galacto-oligosaccharide samples with different concentrations relative to the physiological saline group were calculated. As shown in FIG. 2, different concentrations of sulfate gluco-galacto-oligosaccharide samples had a significant impact on APTT, such that the sulfate gluco-galacto-oligosaccharide could act on the intrinsic coagulation pathway.

**Example 4**

In vivo antithrombotic effects of sulfate gluco-galacto-oligosaccharide

1. Establishment of animal models:

The experimental animals were male C57/BL-6J mice, weighing 18 g to 20 g. The mice were housed under standard experimental conditions with a 12-hour light-12-hour dark cycle with free access to water and food. The mice were anesthetized by intraperitoneal injection of 5% chloral hydrate, and administration of the drug was administered through the tail vein after confirming that the mice were completely anesthetized and had no eyelid reflex. The mice were randomly divided into three groups. A first group was a negative control group and was injected with physiological saline through the tail vein; a second group was a low-dose group, with 0.5 mg/mL sulfate gluco-galacto-oligosaccharide injected into the tail vein; and a third group was a high-dose group, with 1.0 mg/mL sulfate gluco-galacto-oligosaccharide injected into the tail vein. After tail vein administration, the mouse carotid arteries were dissected, and a filter paper piece (1 mm$\times$2 mm) dipped in 6.0% ferric chloride solution was applied to the outer wall of the mouse carotid artery to allow induction for 3 min to construct a ferric chloride-induced carotid artery thrombosis model.

2. Determination of blood vessel occlusion time

The blood flow and thrombosis of the mouse carotid arteries were observed using a laser speckle flow imager, and the mouse carotid arteries were observed under the moorFLPI-2 lens for 15 min. The carotid arteries of the mice at the end of the observation were carefully dissected and stained with HE.

3. Data analysis

Charts were produced using GraphPad Prism 6.0 software, and statistical differences were analyzed using one-way analysis of variance and Dunnett's multiple comparisons. $P<0.05$ indicated a significant difference.

Blood vessel occlusion time refers to a duration from removal of the filter paper soaked in ferric chloride to the blood vessel occlusion. If no thrombosis was observed 15 min after removal of the ferric chloride filter paper, 15 min was considered as the vessel occlusion time. Combined with FIG. 3, in the negative control group (physiological saline group), the average blood vessel occlusion time was $4.5\pm0.23$ min. Under the dosages of 0.5 mg/mL and 1.0 mg/mL of sulfate gluco-galacto-oligosaccharide in the sample, the average blood vessel occlusion times were $5.4\pm1.37$ min and 15 min, respectively. Blood flow remained unchanged at a dose of 1.0 mg/mL, indicating that the sulfate gluco-galacto-oligosaccharide had significant antithrombotic effects.

**Example 5**

Determination of bleeding risk with sulfate gluco-galacto-oligosaccharide

1. Establishment of animal models:

The experimental animals were male C57/BL-6J mice, weighing 18 g to 20 g. The mice were housed under standard experimental conditions with a 12-hour light-12-hour dark cycle with free access to water and food. The mice were anesthetized by intraperitoneal injection of 5% chloral hydrate, and administration of the drug was administered through the tail vein after confirming that the mice were completely anesthetized and had no eyelid reflex. The mice were randomly divided into three groups. A first group was a negative control group and was injected with physiological saline through the tail vein; a second group was a low-dose group, with 0.5 mg/mL sulfate gluco-galacto-oligosaccharide injected into the tail vein; and a third group was a high-dose group, with 1.0 mg/mL sulfate gluco-galacto-oligosaccharide injected into the tail vein.

2. Determination of tail tip bleeding time:

10 min after administration, the mouse was placed stably with its tail in a naturally straightened state. The mouse tail was cut off 3 mm from the tip, and then immersed in a silanized tube filled with 37°C physiological saline. Start timing from cutting off the tail tip, 20 min. the accumulated bleeding time t after cutting off the tail tip of the mouse was recorded with a stopwatch. If the bleeding time t was greater than 20 min, it was recorded as 20 min.

3. Data analysis

Data processing was conducted using the Graphpad Prism 6.0 software. A dot plot of sample concentration was made versus bleeding time. $P<0.05$ indicated a significant difference.

Bleeding time refers to a duration required from bleeding to natural hemostasis after capillary damage. In the negative control group (physiological saline group), the cumulative bleeding time was $10.35\pm5.48$ min. At the dosages of 0.5 mg/mL and 1.0 mg/mL of sulfate gluco-galacto-oligosaccharide, the cumulative bleeding times were $11.88\pm4.44$ min and $11.60\pm4.13$ min, respectively, with no statistically significant difference ($P>0.05$). Combined with FIG. 4, it indicated that the sulfate gluco-galacto-oligosaccharide had a significant anti-thrombotic activity without obvious bleeding risk.

The above description is a general description of the present disclosure. Changes in form and substitutions of equivalent value may be made according to circumstances or actual needs. Various changes and modifications may be made on the present disclosure by those skilled in the prior art, and these equivalent forms also fall within the scope defined by the present disclosure.

## Claims

1. An anticoagulant and/or antithrombotic drug, wherein the anticoagulant and/or antithrombotic drug comprises sulfate gluco-galacto-oligosaccharide and the sulfate gluco-galacto-oligosaccharide has a structural formula shown in formula 1:

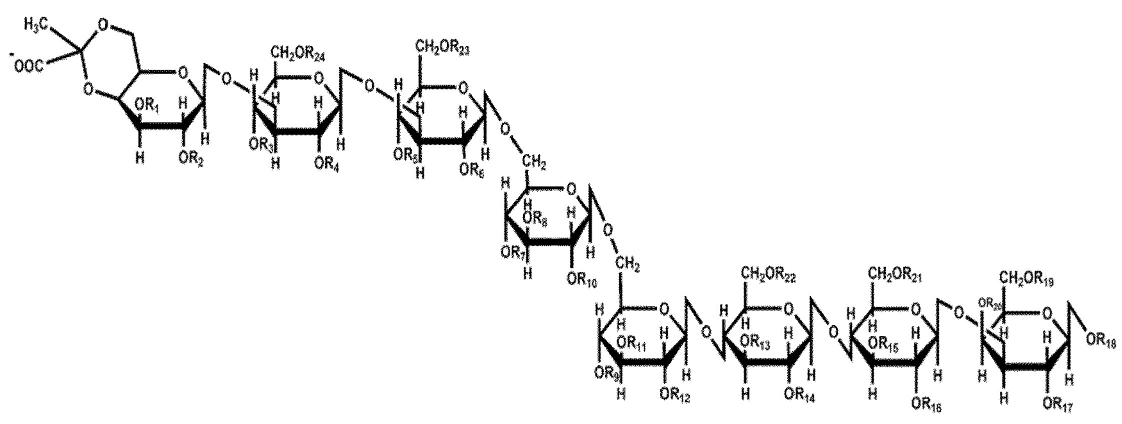

formula 1,

wherein, $R_1$, $R_2$, $R_3$, $R_4$, $R_5$, $R_6$, $R_7$, $R_8$, $R_9$, $R_{10}$, $R_{11}$, $R_{12}$, $R_{13}$, $R_{14}$, $R_{15}$, $R_{16}$, $R_{17}$, $R_{18}$, $R_{19}$, $R_{20}$, $R_{21}$, $R_{22}$, $R_{23}$, and $R_{24}$ independently are selected from the group consisting of $SO_3^-$ and H, but are not all H, and wherein the sulfate gluco-galacto-oligosaccharide has an average of 0.5 to 3 $SO_3$-on each sugar residue.

**2.** The drug according to claim 1 for use in the treatment of cerebral infarction, atherosclerotic plaque formation in a carotid artery, pulmonary infarction, myocardial infarction, splenic infarction, thrombosis in a mesenteric vein, mesenteric arterial embolism, a venous thromboembolic disease, lower extremity arteriosclerosis obliterans; and disseminated intravascular coagulation or for use as an anticoagulant of a blood sample specimen for blood transfusion, extracorporeal hemodialysis, or peritoneal dialysis.

**3.** The drug according to claim 1 or 2, wherein the sulfate gluco-galacto-oligosaccharide has an average of 0.5 to 2.5 $SO_3^-$ on each sugar residue.

**4.** The drug according to claim 3, wherein the sulfate gluco-galacto-oligosaccharide has an average of 2.1 to 2.5 $SO_3^-$ on each sugar residue.

**5.** The drug according to claim 1, wherein a preparation method of the sulfate gluco-galacto-oligosaccharide comprises: subjecting gluco-galacto-oligosaccharide to sulfation with a sulfating reagent to obtain the sulfate gluco-galacto-oligosaccharide.

**6.** The drug according to claim 5, wherein the sulfating reagent is a mixture of pyridine and chlorosulfonic acid.

**7.** The drug according to claim 5, wherein the preparation method of the sulfate gluco-galacto-oligosaccharide specifically comprises: preparing the sulfating reagent using pyridine and chlorosulfonic acid; adding a dimethyl sulfoxide (DMSO) solution of the gluco-galacto-oligosaccharide into the sulfating reagent under stirring to allow the sulfation in an oil bath at 50°C to 120°C; and subjecting an obtained reaction solution to a post-treatment and purification to obtain the sulfate gluco-galacto-oligosaccharide.

**8.** The drug according to claim 7, wherein the pyridine and the chlorosulfonic acid in the mixture are at a volume ratio of 3:1 to 8:1.

**9.** The drug according to claim 7, wherein the gluco-galacto-oligosaccharide and DMSO in the DMSO solution of the gluco-galacto-oligosaccharide are at a mass-to-volume ratio of 1 g: (10-20) mL.

**10.** The drug according to any one of claims 5 to 7, wherein the gluco-galacto-oligosaccharide and the sulfating reagent are at a mass-to-volume ratio of 1 g: 20 mL to 1 g: 60 mL.

**11.** The drug according to claim 7, wherein the sulfation is conducted at 60°C for 2 h to 5 h.

**12.** The drug according to claim 7, wherein the post-treatment and the purification comprise: adjusting the reaction solution to a neutral pH value with a 15% NaOH solution, conducting centrifugation at 8,000 rpm and 4°C to remove insoluble impurities, collecting an obtained supernatant to allow dialysis with a dialysis bag of 500 Da to 1,000 Da, subjecting a resulting dialyzate to concentration and desalination using an organic ultrafiltration membrane, and then conducting freeze-drying or ethanol precipitation to obtain the sulfate gluco-galacto-oligosaccharide.

## Patentansprüche

**1.** Antikoagulatorischer und/oder antithrombotischer Arzneistoff, wobei der antikoagulatorische und/oder der antithrombotische Arzneistoff Sulfatglucogalactooligosaccharid umfassen/umfasst und das Sulfatglucogalactooligosaccharid eine in Formel 1 gezeigte Strukturformel aufweist:

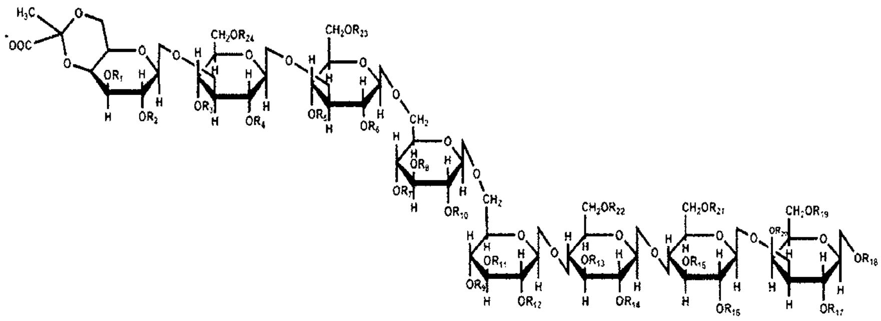

Formel 1,

wobei $R_1$ ,$R_2$, $R_3$, $R_4$, $R_5$, $R_6$, $R_7$, $R_8$, $R_9$, $R_{10}$, $R_{11}$, $R_{12}$, $R_{13}$, $R_{14}$, $R_{15}$, $R_{16}$, $R_{17}$, $R_{18}$, $R_{19}$, $R_{20}$, $R_{21}$, $R_{22}$, $R_{23}$ und $R_{24}$ unabhängig aus der Gruppe ausgewählt sind, die aus $SO_3^-$ und H besteht, jedoch nicht alle H sind, und wobei das Sulfatglucogalactooligosaccharid durchschnittlich 0,5 bis 3 $SO_3$- an jedem Zuckerrest aufweist.

**2.** Arzneistoff nach Anspruch 1 zur Verwendung bei der Behandlung eines Hirninfarkts, einer atherosklerotischen Plaquebildung in einer Halsschlagader, eines Lungeninfarkts, eines Herzinfarkts, eines Milzinfarkts, einer Thrombose in einer Mesenterialvene, einer mesenterialen arteriellen Embolie, einer venösen thromboembolischen Erkrankung, einer Arteriosklerose obliterans der unteren Extremitäten; und disseminierte intravasale Gerinnung oder zur Verwendung als Antikoagulans einer Blutprobe für Bluttransfusionen, extrakorporale Hämodialyse oder Peritonealdialyse.

**3.** Arzneistoff nach Anspruch 1 oder 2, wobei das Sulfatglucogalactooligosaccharid durchschnittlich 0,5 bis 2,5 $SO_3^-$ an jedem Zuckerrest aufweist.

**4.** Arzneistoff nach Anspruch 3, wobei das Sulfatglucogalactooligosaccharid durchschnittlich 2,1 bis 2,5 $SO_3^-$ an jedem Zuckerrest aufweist.

**5.** Arzneistoff nach Anspruch 1, wobei ein Herstellungsverfahren des Sulfatglucogalactooligosaccharids Folgendes umfasst: Unterziehen des Glucogalactooligosaccharids einer Sulfatierung mit einem Sulfatierungsreagenz, um das Sulfatglucogalactooigosaccharid zu erhalten.

**6.** Arzneistoff nach Anspruch 5, wobei das Sulfatierungsreagenz ein Gemisch aus Pyridin und Chlorsulfonsäure ist.

**7.** Arzneistoff nach Anspruch 5, wobei das Herstellungsverfahren des Sulfatglucogalactooligosaccharids insbesondere Folgendes umfasst: Herstellen des Sulfatierungsreagenzes unter Verwendung von Pyridin und Chlorsulfonsäure; Zusetzen einer Dimethylsulfoxid (DMSO)-Lösung des Glucogalactooligosaccharids in das Sulfatierungsreagenz unter Rühren, um die Sulfatierung in einem Ölbad bei 50 °C bis 120 °C zu ermöglichen; und Unterziehen einer erhaltenen Reaktionslösung einer Nachbehandlung und Reinigung, um das Sulfatglucogalactooligosaccharid zu erhalten.

**8.** Arzneistoff nach Anspruch 7, wobei das Pyridin und die Chlorsulfonsäure in dem Gemisch in einem Volumenverhältnis von 3:1 bis 8:1 vorliegen.

**9.** Arzneistoff nach Anspruch 7, wobei das Glucogalactooligosaccharid und DMSO in der DMSO-Lösung des Glucogalactooligosaccharids in einem Masse-Volumen-Verhältnis von 1 g: (10-20) ml vorliegen.

**10.** Arzneistoff nach einem der Ansprüche 5 bis 7, wobei das Glucogalactooligosaccharid und das Sulfatierungsreagenz in einem Masse-Volumen-Verhältnis von 1 g: 20 ml bis 1 g: 60 ml vorliegen.

**11.** Arzneistoff nach Anspruch 7, wobei die Sulfatierung bei 60 °C für 2 h bis 5 h durchgeführt wird.

**12.** Arzneistoff nach Anspruch 7, wobei die Nachbehandlung und die Reinigung Folgendes umfassen: Einstellen der Reaktionslösung auf einen neutralen pH-Wert mit einer 15 % NaOH-Lösung, Durchführen einer Zentrifugation bei 8.000 U/min und 4 °C, um unlösliche Verunreinigungen zu entfernen, Sammeln eines erhaltenen Überstandes, um eine Dialyse mit einem Dialysebeutel von 500 Da bis 1.000 Da zu ermöglichen, Unterziehen eines resultierenden

Dialysats einer Einengung und Entsalzung unter Verwendung einer organischen Ultrafiltrationsmembran, und dann Durchführen einer Gefriertrocknung oder Ethanolfällung, um das Sulfatglucogalactooligosaccharid zu erhalten.

## Revendications

1. Médicament anticoagulant et/ou antithrombotique, dans lequel le médicament anticoagulant et/ou antithrombotique comprend un gluco-galacto-oligosaccharide sulfaté, et le gluco-galacto-oligosaccharide sulfaté ayant une formule développée représentée par la formule 1 :

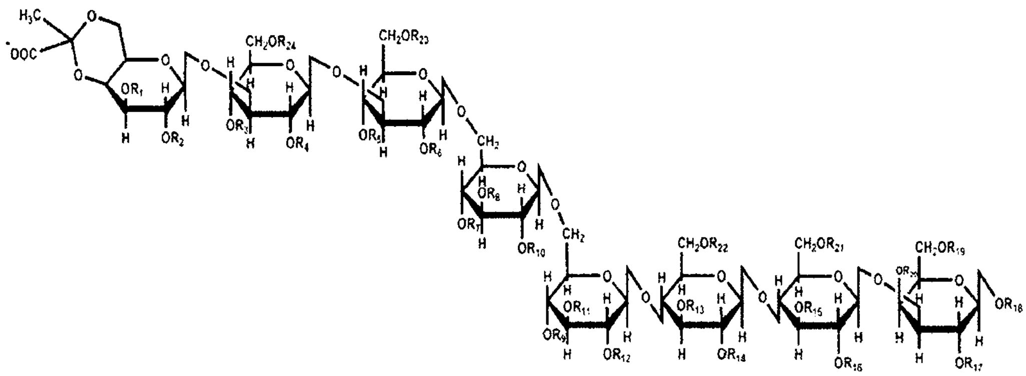

formule 1,

dans laquelle $R_1$, $R_2$, $R_3$, $R_4$, $R_5$, $R_6$, $R_7$, $R_8$, $R_9$, $R_{10}$, $R_{11}$, $R_{12}$, $R_{13}$, $R_{14}$, $R_{15}$, $R_{16}$, $R_{17}$, $R_{18}$, $R_{19}$, $R_{20}$, $R_{21}$, $R_{22}$, $R_{23}$ et $R_{24}$ sont indépendamment choisis dans le groupe constitué par $SO_3^-$ et H, mais ne représentent pas tous H, et dans laquelle le gluco-galacto-oligosaccharide sulfaté a en moyenne de 0,5 à 3 $SO_3$- sur chaque résidu de sucre.

2. Médicament selon la revendication 1, destiné à être utilisé dans le traitement de l'infarctus cérébral, de la formation de plaques d'athérosclérose dans une artère carotide, de l'infarctus pulmonaire, de l'infarctus du myocarde, de l'infarctus splénique, de la thrombose dans une veine mésentérique, de l'embolie artérielle mésentérique, d'une maladie thromboembolique veineuse, de l'artériosclérose oblitérante des membres inférieurs ; et de la coagulation intravasculaire disséminée, ou destiné à être utilisé comme anticoagulant d'un échantillon de sang pour une transfusion sanguine, une hémodialyse extracorporelle ou une dialyse péritonéale.

3. Médicament selon la revendication 1 ou 2, dans lequel le gluco-galacto-oligosaccharide sulfaté a en moyenne de 0,5 à 2,5 $SO_3^-$ sur chaque résidu de sucre.

4. Médicament selon la revendication 3, dans lequel le gluco-galacto-oligosaccharide sulfaté a en moyenne de 2,1 à 2,5 $SO_3^-$ sur chaque résidu de sucre.

5. Médicament selon la revendication 1, dans lequel un procédé de préparation du gluco-galacto-oligosaccharide sulfaté consiste à : soumettre le gluco-galacto-oligosaccharide à une sulfatation avec un réactif de sulfatation pour obtenir le gluco-galacto-oligosaccharide sulfaté.

6. Médicament selon la revendication 5, dans lequel le réactif de sulfatation est un mélange de pyridine et d'acide chlorosulfonique.

7. Médicament selon la revendication 5, dans lequel le procédé de préparation du gluco-galacto-oligosaccharide sulfaté consiste spécifiquement à : préparer le réactif de sulfatation à l'aide de pyridine et d'acide chlorosulfonique ; ajouter une solution de diméthylsulfoxyde (DMSO) du gluco-galacto-oligosaccharide au réactif de sulfatation sous agitation pour permettre la sulfatation dans un bain d'huile à une température de 50 °C à 120 °C ; et soumettre la solution réactionnelle obtenue à un post-traitement et à une purification pour obtenir le gluco-galacto-oligosaccharide sulfaté.

8. Médicament selon la revendication 7, dans lequel la pyridine et l'acide chlorosulfonique dans le mélange sont présents à un rapport volumique de 3:1 à 8:1.

9. Médicament selon la revendication 7, dans lequel le gluco-galacto-oligosaccharide et le DMSO dans la solution de DMSO du gluco-galacto-oligosaccharide sont présents à un rapport masse/volume de 1 g : (10-20) mL.

10. Médicament selon l'une quelconque des revendications 5 à 7, dans lequel le gluco-galacto-oligosaccharide et le réactif de sulfatation sont présents à un rapport masse/volume de 1 g : 20 mL à 1 g : 60 mL.

11. Médicament selon la revendication 7, dans lequel la sulfatation est réalisée à 60 °C pendant de 2 h à 5 h.

12. Médicament selon la revendication 7, dans lequel le post-traitement et la purification consistent à : ajuster la solution réactionnelle à une valeur de pH neutre avec une solution de NaOH à 15 %, réaliser une centrifugation à 8 000 tr/min et 4 °C pour éliminer les impuretés insolubles, collecter un surnageant obtenu pour permettre une dialyse avec une poche de dialyse de 500 Da à 1 000 Da, soumettre un dialysat ainsi obtenu à une concentration et à un dessalement en utilisant une membrane d'ultrafiltration organique, et puis réaliser une lyophilisation ou une précipitation à l'éthanol pour obtenir le gluco-galacto-oligosaccharide sulfaté.

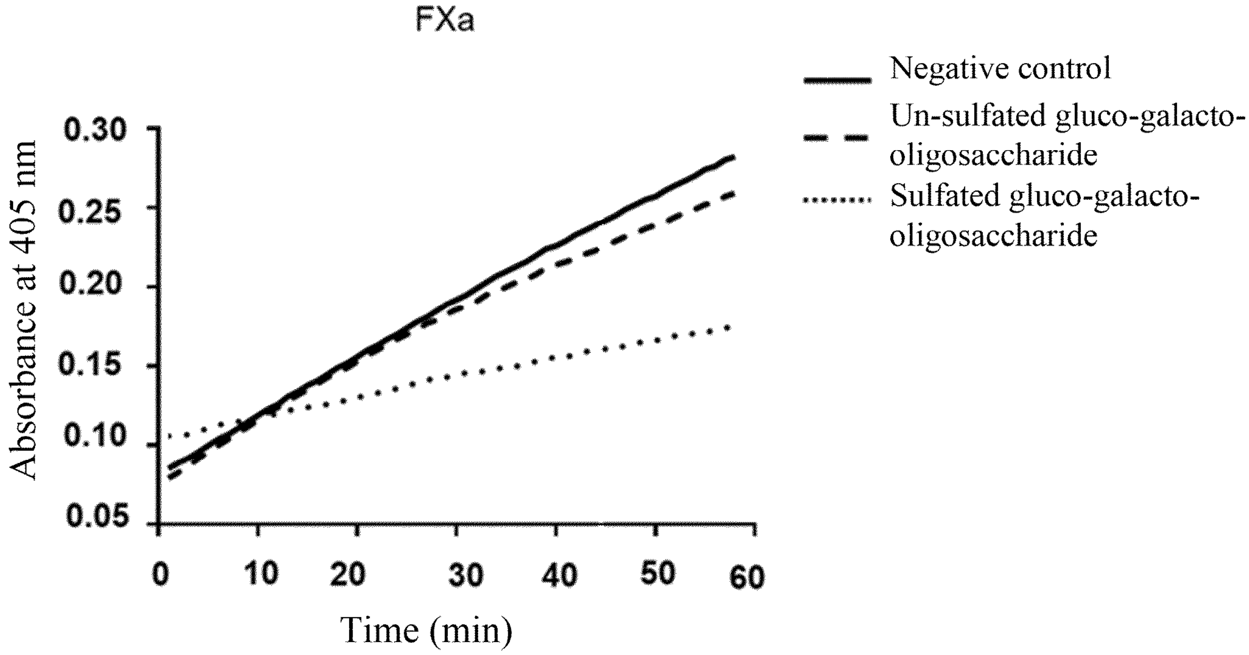
FXa
Negative control
Un-sulfated gluco-galacto-oligosaccharide
Sulfated gluco-galacto-oligosaccharide
Absorbance at 405 nm
0.30
0.25
0.20
0.15
0.10
0.05
0
10
20
30
40
50
60
Time (min)

**FIG. 1**

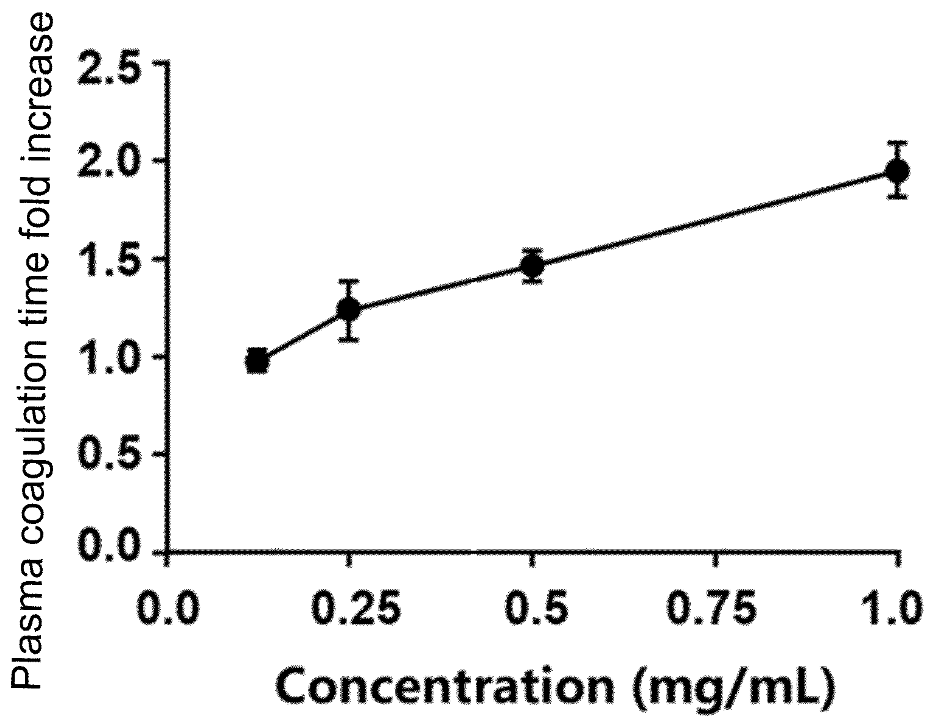
Plasma coagulation time fold increase
2.5
2.0
1.5
1.0
0.5
0.0
0.0
0.25
0.5
0.75
1.0
Concentration (mg/mL)

**FIG. 2**

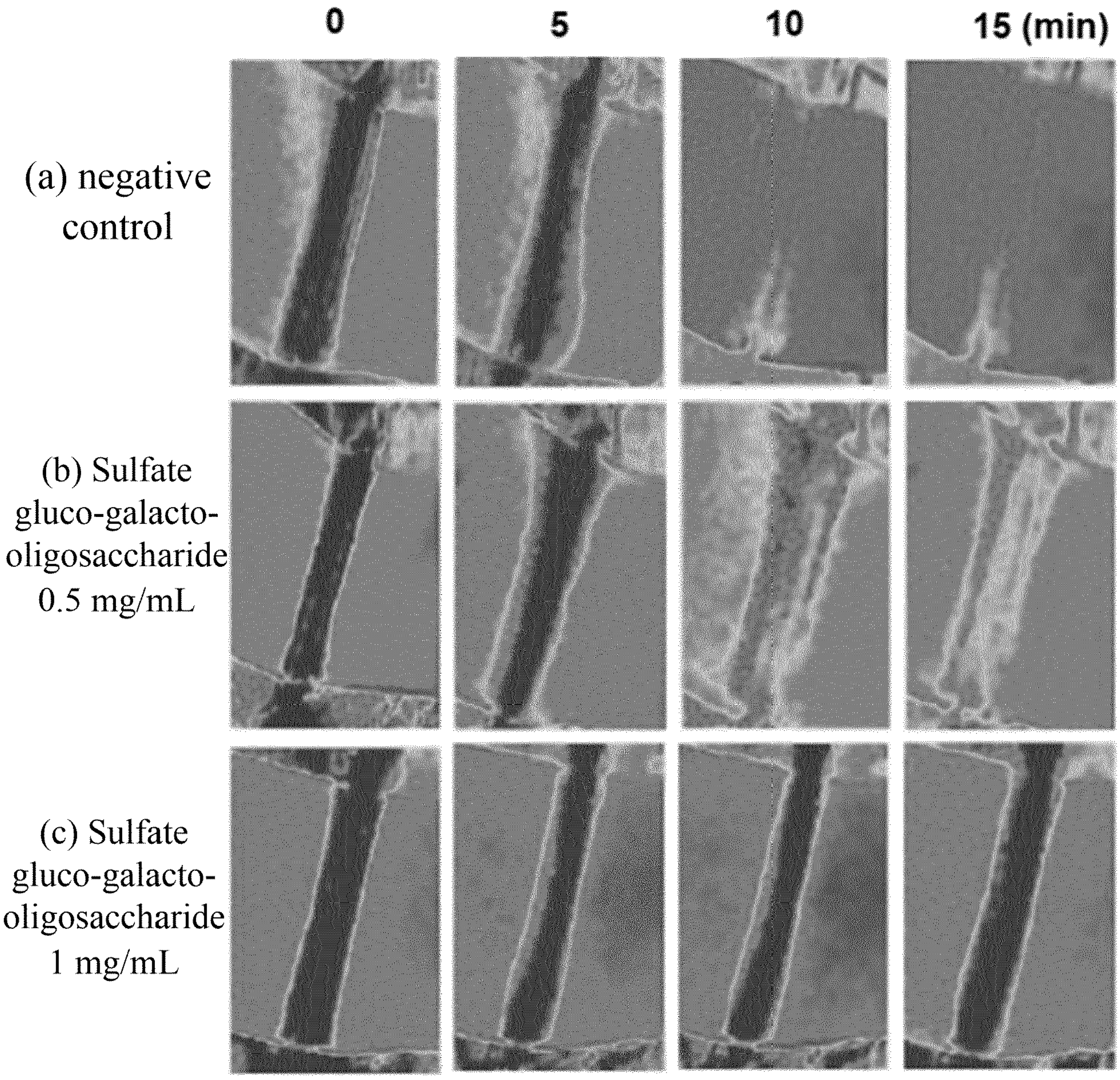
0
5
10
15 (min)
(a) negative control
(b) Sulfate gluco-galacto-oligosaccharide 0.5 mg/mL
(c) Sulfate gluco-galacto-oligosaccharide 1 mg/mL

FIG. 3

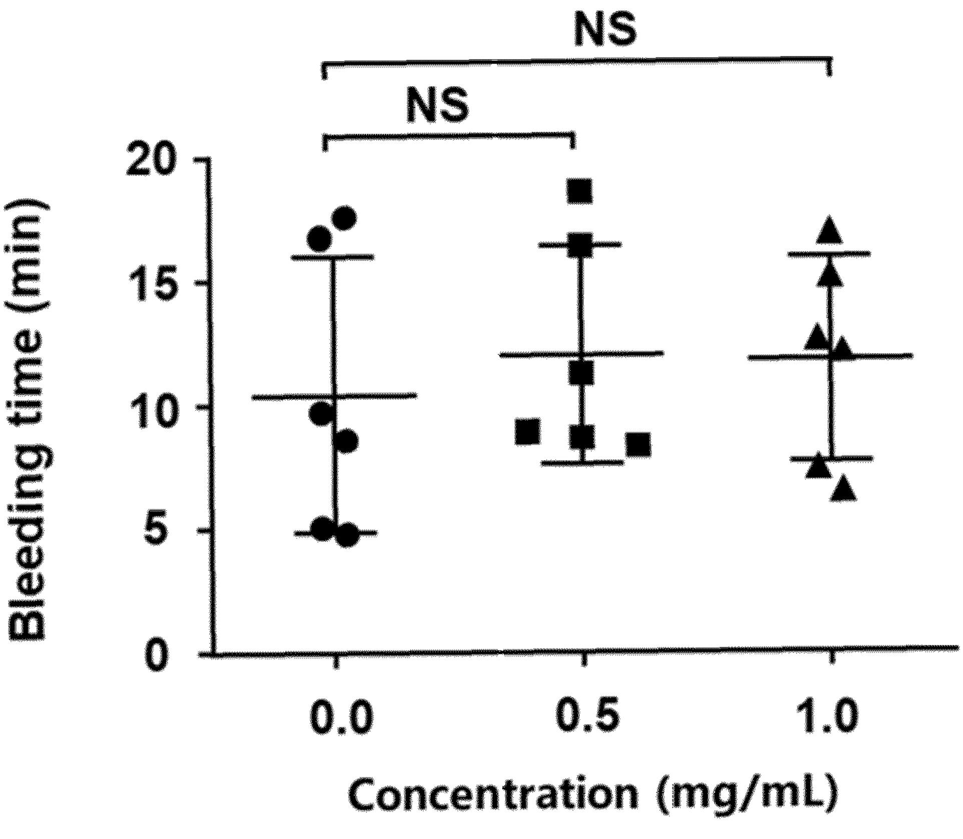
NS
NS
20
15
10
5
0
Bleeding time (min)
0.0
0.5
1.0
Concentration (mg/mL)

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6143730 A
- CN 1251992 A
- CN 101209260 A
- WO 2019090203 A1


### Non-patent literature cited in the description


- **MARTINICHEN-HERRERO et al.** Anticoagulant and antithrombotic activities of a chemically sulfated galactoglucomannan obtained from the lichen Cladonia ibitipocae. *International Journal of Biological Macromolecules*, vol. 35, 97-102
- **WANG et al.** Preparation and Gut Microbiota Modulatory Property of the Oligosaccharide Riclinoctaose. *Journal of Agricultural and Food Chemistry*, vol. 69, 3667-3676
- **WANG, L.** ; **CHENG, R.** ; **SUN, X. et al.** Preparation and Gut Microbiota Modulatory Property of the Oligosaccharide Riclinoctaose. *J. Agric. Food Chem.*, 31 March 2021, vol. 69 (12), 3667-3676
- **WANG, L.** ; **CHENG, R.** ; **SUN, X. et al.** *J. Agric. Food Chem.*, 2021, vol. 69, 3667-3676